# EUROPÄISCHE PATENTSCHRIFT

(11) **EP 1 682 021 B1**
(45) Veröffentlichungstag und Bekanntmachung des Hinweises auf die Patenterteilung: **23.02.2011**
(21) Anmeldenummer: 04802663.7
(22) Anmeldetag: 29.10.2004
(51) Int. Cl.: A61B 17/86

(54) **KNOCHENSCHRAUBE**
BONE SCREW
VIS A OS

(30) Priorität: 29.10.2003 DE 10350424
(43) Veröffentlichungstag der Anmeldung: 26.07.2006
(73) Patentinhaber: Ulrich GmbH & Co. KG, 89081 Ulm (DE)
(72) Erfinder: WILLMANN, Niko, 89077 Ulm (DE)
(74) Vertreter: Hentrich, Swen
(86) Internationale Anmeldenummer: PCT/DE2004/002417
(87) Internationale Veröffentlichungsnummer: WO 2005/041798

(56) Entgegenhaltungen:
- WO-A-93/06786
- US-A- 5 334 204
- US-A1- 2002 055 783
- US-B1- 6 679 701

## Beschreibung

Die Erfindung betrifft eine Knochenschraube, insbesondere Spongiosaschraube, mit einem Schraubenkopf, einem Schraubenschaft und einem in Gewindegängen auf dem Schraubenschaft ausgebildeten Gewinde, wobei ausgehend von dem freien Ende des Schraubenschaftes eine Mehrzahl von ersten Gewindegängen mit glatten Schneidkanten in Richtung des Schraubenkopfes verlaufen, wobei an die ersten Gewindegänge in Richtung von dem Schraubenkopf gestaffelt anschliessend zweite Gewindegänge ausgebildet sind mit in den Schneidkanten ausgebildeten Freischneidungen, und wobei die Freischneidungen der zweiten Gewindegänge, bezogen auf die Bogenlänge in Umfangsrichtung, gegenüber dem vom freien Ende, jeweils vorangehenden zweiten Gewindegang vergrössert sind.

Knochenschrauben sind aus der Praxis bekannt, die in der Medizin dazu verwendet werden, um Knochen nach einer Fraktur wieder zusammen zu fügen, Knochenplatten zu befestigen, oder, im Falle von Spongiosaschrauben, Implantate an Wirbelkörpern zu fixieren. Derartige Knochenschrauben verbleiben häufig dauerhaft im Körper des Patienten und müssen daher für einen langen Zeitraum die ihnen zugedachte Funktion ausführen, ohne sich dabei unter Belastungen wieder zu lösen.

Eine Knochenschraube der eingangs genannten Art ist in der US 2002/0055783 A1 offenbart, bei der die Freischneidungen symmetrisch gestaltet sind und im wesentlichen beim Einschrauben nur als Unterbrechung der Schneidkanten wirken, langfristig aber ein Einwachsen von Knochen und damit einen Forschluß ermöglichen.

Der Erfindung liegt daher die Aufgabe zugrunde, eine Knochenschraube der eingangs genannten Art so auszubilden, dass nach deren Einschrauben unerwünschte Lageänderungen, insbesondere ein sich Lösen verhindert ist.

Diese Aufgabe wird nach der Erfindung bei einer Knochenschraube der eingangs genannten Art dadurch gelöst, dass die Freischneidung (9) nach Art einer Sehne von den Schneidkanten (7) der zweiten Gewindegänge (8) und der dritten Gewindegänge (10) in Richtung des Schraubenschaftes (3) verläuft und in einem Bogen wieder an die Schneidkante (7) anschliesst, so dass Widerhaken (11) gebildet sind, die einer Rückdrehung einen Widerstand entegen setzen.

Bei einer derartigen Knochenschraube ist sichergestellt, dass bei deren Eindrehen in den Knochen durch die ersten Gewindegänge bereits auf kurzer Führungslänge ein guter Halt der Knochenschraube im Knochen erreicht wird unter Gewährleistung eines sauberen Schnittes durch die glatten Schneidkanten der ersten Gewindegänge, der von den nachfolgenden Schneidkanten der zweiten und dritten Gewindegänge durchlaufen werden kann. Die Freischneidungen der zweiten und dritten Gewindegänge bewirken dabei nach Art der Widerhaken ein festeres Verkrallen des Schraubenschaftes in den Knochen und gewährleisten, dass nach dem Eindrehen der Knochenschraube Körpergewebe, also Knochenmaterial, in den Schneidgang im Bereich der Freischneidungen einsprossen kann und so eine Blockade darstellt gegen das Lösen der Knochenschraube durch Rückdrehung des Gewindes in dem Schneidkanal, also ein besonders fester Sitz der Knochenschraube im Knochen erreicht wird.

Im Rahmen der Erfindung sind an die zweiten Gewindegänge gestaffelt anschliessend dritte Gewindegänge mit Freischneidungen in konstanter Grösse angeordnet sind. Dadurch wird der Vorteil erreicht, dass in einfacher Weise durch eine Variation der Anzahl der dritten Gewindegänge die Länge der Knochenschraube variiert werden kann, ohne dass durch Fortschreiten der Länge durch die zunehmende Ausweitung der Freischneidungen der zweiten Gewindegänge die Schneidkanten sich im Bereich des Schraubenkopfes so verkürzen, dass deren Funktion gefährdet ist.

Als günstig hat es sich erwiesen, wenn in jedem zweiten Gewindegang und in jedem dritten Gewindegang auf einem Bogen von 360 mehrere Freischneidungen, vorzugsweise drei Freischneidungen ausgebildet sind, um so einen festen Sitz der Knochenschraube zu erzielen, und, bei gleichmässiger Verteilung der Freischneidungen um den Umfang des Schraubenschaftes, eine gleichmässige Drehbewegung der Knochenschraube bei deren Einschrauben zu ermöglichen.

Im Rahmen der Erfindung besteht die Möglichkeit, dass der Schraubenschaft zylindrisch geformt ist, wodurch besonders deutlich der Nutzen der ersten Gewindegänge ausgeprägt ist, die den Schraubenkanal schaffen, durch den von der Oberfläche des Knochens auch die zweiten Gewindegänge und die dritten Gewindegänge eingeschraubt werden können. Es besteht allerdings auch die Möglichkeit, dass der Schraubenschaft konisch geformt ist, um zu erreichen, dass auch die zweiten Gewindegänge und dritten Gewindegänge sich selber in das Knochenmaterial einarbeiten und so einen festen Sitz gewährleisten. Um die den ersten Gewindegängen zugedachte Funktion auszuführen, ist es ausreichend, wenn die ersten Gewindegänge drei Gewindegänge umfassen. Ausreichend ist es auch, wenn die zweiten Gewindegänge drei Gewindegänge umfassen.

Bei Schrauben mit einem konisch geformten Schraubenschaft bietet es sich an, dass die Länge der Schneidkanten der zweiten Gewindegänge konstant ist, dass also die Vergrösserung der Umfangslänge der näher an dem Schraubenkopf liegenden Gewindegänge den Freischneidungen zugeordnet wird, um so trotz des gegebenen Nutzens durch grösser werdende Freischneidungen eine gute Führung durch ausreichend lange Schneidkanten zu haben.

Im folgenden wird die Erfindung an einem in der Zeichnung dargestellten Ausführungsbeispiel näher erläutert; es zeigen :

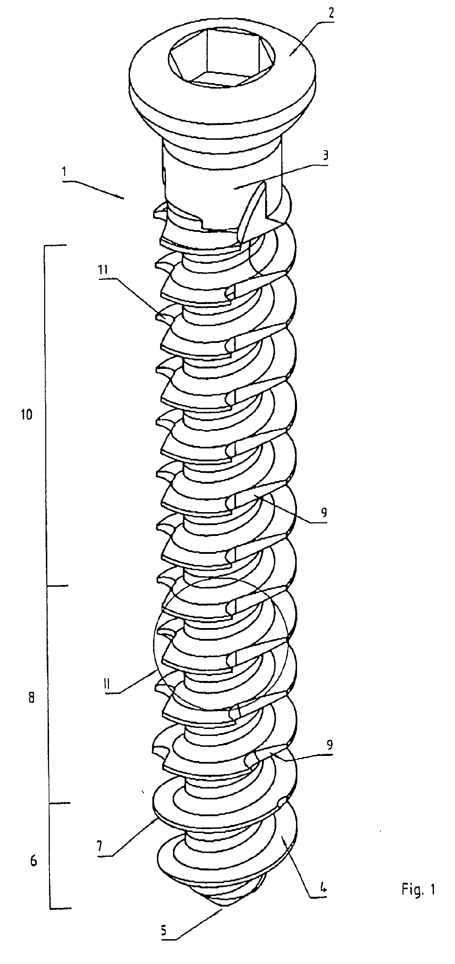

- Fig. 1: eine perspektivische Darstellung einer Knochenschraube,
- Fig. 2: das Detail II aus Fig. 1, Fig. 3 eine perspektivische Darstellung der Knochenschraube aus
- Fig. 3: aus einem anderen Blickwinkel,

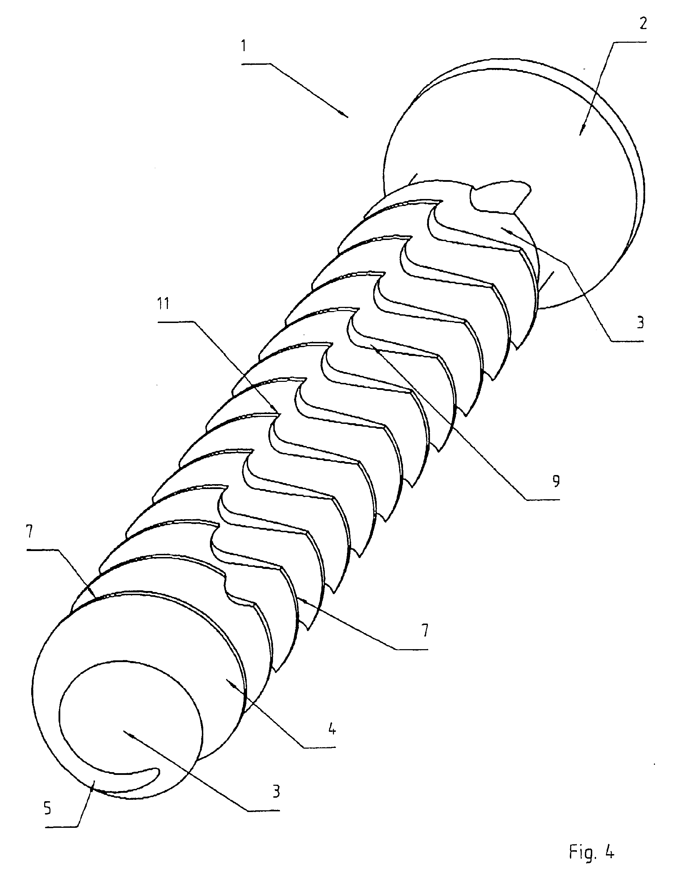

- Fig.4: eine perspektivische Darstellung der Knochenschraube aus Fig. 1 aus einem nochmals anderen Blickwinkel, und
- Fig. 5: der Schnitt V-V aus Fig. 1.

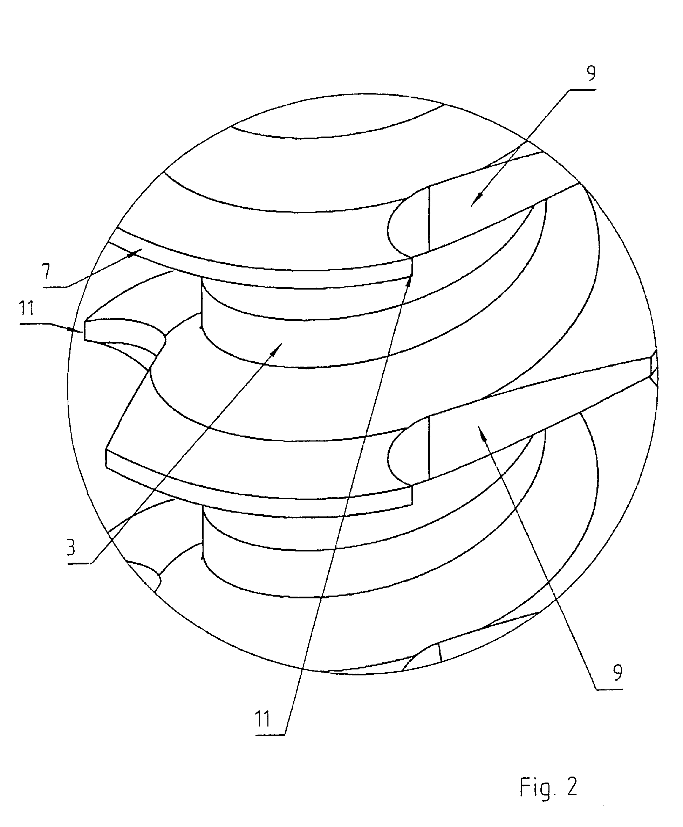

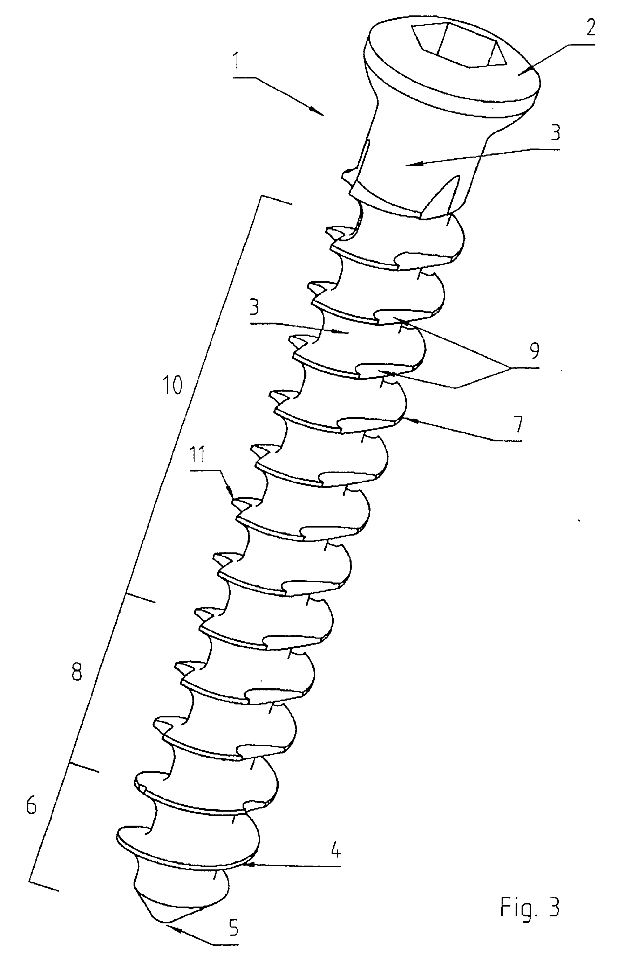

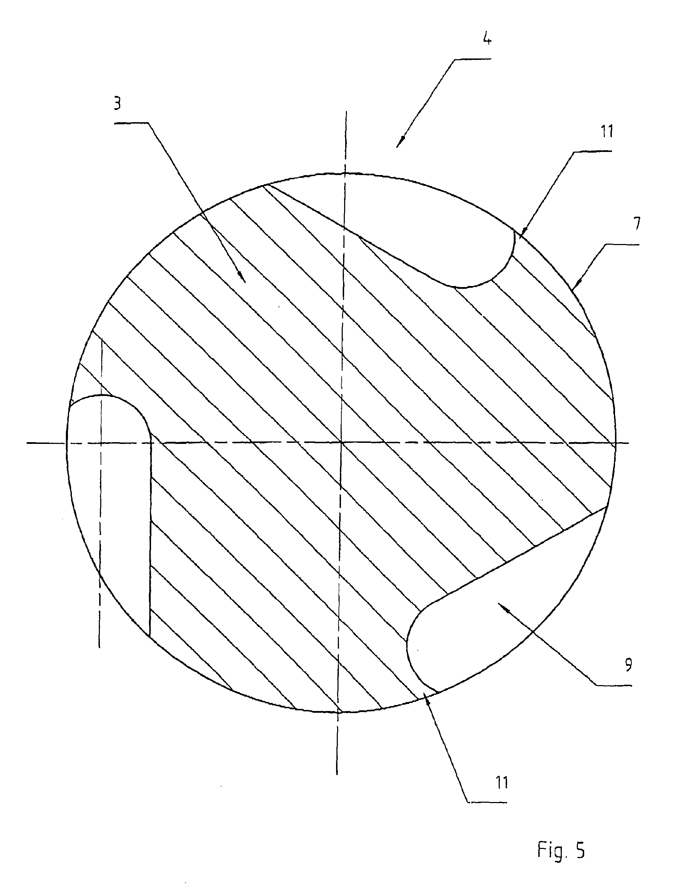

In der Zeichnung ist eine Knochenschraube 1 dargestellt, die aus einem Schraubenkopf 2, einem Schraubenschaft 3 und einem mit Gewindegängen auf dem Schraubenschaft 3 ausgebildeten Gewinde 4 besteht. Ausgehend von dem freien Ende 5 des Schraubenschaftes 3 sind mehrere Gewindegänge zu ersten Gewindegängen 6 zusammengefasst, deren Schneidkanten 7 glatt sind. An die ersten Gewindegänge 6 anschliessend in Richtung des Schraubenkopfes 2 gestaffelt sind zweite Gewindegänge 8 ausgebildet, bei denen in den Schneidkanten 7 Freischneidungen 9 eingebracht sind, und zwar auf einem Bogen von 360 drei gleichmässig über den Umfang angeordnete Freischneidungen 9. Insbesondere aus Fig. 3 ist dabei ersichtlich, dass die Freischneidungen 9 der zweiten Gewindegänge 8, bezogen auf die Bogenlänge in Umfangsrichtung, gegenüber den jeweils von dem freien Ende 5 des Schraubenschaftes 3 vorangehenden zweiten Gewindegang 8 vergrössert sind, also bei den zweiten Gewindegängen 8 sich die Bogenlänge der Schneidkanten 7 in Richtung des Schraubenkopfes 2 zugunsten einer Zunahme der Länge der Freischneidungen 9 verkürzt im Vergleich zu Schneidkanten mit nicht vergrößerten Freischneidungen. Fig. 3 lässt auch erkennen, dass an die zweiten Gewindegänge 8 gestaffelt anschliessend dritte Gewindegänge 10 angeordnet sind, deren Freischneidungen 9 konstant verbleiben. Die Detaildarstellung in Fig. 2 sowie der Schnitt aus Fig. 5 lassen erkennen, dass die Freischneidungen 9 nach Art einer Sehne von den Schneidkanten 7 der zweiten Gewindegänge 8 und der dritten Gewindegänge 10 in Richtung des Schraubenschaftes 3 verlaufen und in einem Bogen wieder an die Schneidkante 7 anschliesst, so dass Widerhaken 11 gebildet sind, die einer Rückdrehung der Knochenschraube 1 einen Widerstand entgegensetzen.

Das in der Zeichnung dargestellte Ausführungsbeispiel weist einen zylindrisch geformten Schraubenschaft 3 auf, wobei aber auch die Möglichkeit besteht, dass der Schraubenschaft 3 konisch geformt ist, wozu es sich dann anbietet, dass die Länge der Schneidkanten 7 der zweiten Gewindegänge 8 konstant ist.

## Patentansprüche

1. Knochenschraube, insbesondere Spongiosaschraube, mit einem Schraubenkopf (2), einem Schraubenschaft (3) und einem in Gewindegängen auf dem Schraubenschaft (3) ausgebildeten Gewinde (4), wobei ausgehend von dem freien Ende (5) des Schraubenschaftes (3) eine Mehrzahl von ersten Gewindegängen (6) mit glatten Schneidkanten (7) in Richtung des Schraubenkopfes (2) verlaufen, wobei an die ersten Gewindegänge (6) in Richtung des Schraubenkopfes (2) gestaffelt anschliessend zweite Gewindegänge (8) ausgebildet sind mit in den Schneidkanten (7) ausgebildeten Freischneidungen (9), und die Freischneidungen (9) der zweiten Gewindegänge (8), bezogen auf die Bogenlänge in Umfangsrichtung, gegenüber dem, vom freien Ende (5), jeweils vorangehenden zweiten Gewindegang (8) vergrossert sind wobei an die zweiten Gewindegänge (8) gestaffelt anschliessend dritte Gewindegänge (10) mit Freischneidungen (9) in konstanter Grösse angeordnet sind, **dadurch gekennzeichnet, dass** die Freischneidung (9) nach Art einer Sehne von den Schneidkanten (7) der zweiten Gewindegänge (8) und der dritten Gewindegänge (10) in Richtung des Schraubenschaftes (3) verläuft und in einem Bogen wieder an die Schneidkante (7) anschliesst, so dass Widerhaken (11) gebildet sind, die einer Rückdrehung einen Widerstand entegen setzen.

2. Knochenschraube nach Anspruch 1 **dadurch gekennzeichnet, dass** in jedem zweiten Gewindegang (8) und in jedem dritten Gewindegang (8) auf einem Bogen von 360 mehrere Freischneidungen (9) ausgebildet sind.

3. Knochenschraube nach Anspruch 2, **dadurch gekennzeichnet, dass** auf einem Bogen von 360 drei Freischneidungen (9) ausgebildet sind.

4. Knochenschrauben nach einem der Ansprüche 1 bis 3, **dadurch gekennzeichnet, dass** der Schraubenschaft (3) zylindrisch geformt ist.

5. Knochenschraube nach einem der Ansprüche 1 bis 3, **dadurch gekennzeichnet, dass** der Schraubenschaft (3) konisch geformt ist.

6. Knochenschraube nach einem der Ansprüche 1 bis 5, **dadurch gekennzeichnet, dass** die ersten Gewindegänge (6) drei Gewindegängen umfassen.

7. Knochenschraube nach einem der Ansprüche 1 bis 6, **dadurch gekennzeichnet, dass** die zweiten Gewindegänge (8) drei Gewindegänge umfassen.

8. Knochenschraube nach einem der Ansprüche 5 bis 7, **dadurch gekennzeichnet, dass** die Länge der Schneidkanten (7) der zweiten Gewindegänge (8) konstant ist.

## Claims

1. A bone screw, in particular a spongiosa screw, comprising a screw head (2), a screw shank (3) and a thread (4) formed in thread flights on the screw shank (3), wherein starting from the free end (5) of the screw shank (3) a plurality of first thread flights (6) with smooth cutting edges (7) extend in the direction of the screw head (2), wherein second thread flights (8) are provided adjoining the first thread flights (6) in stepped relationship in the direction of the screw head (2), with recesses (9) in the cutting edges (7) and the recesses (9) in the second thread flights (8) are increased in size with respect to the arcuate length in the peripheral direction in relation to the respectively preceding second thread flight (8) from the free end (5), wherein third thread flights (10) with recesses (9) of constant size are arranged adjoining the second thread flights (8) in stepped relationship, **characterised in that** the recess (9) extends in the manner of a chord from the cutting edges (7) of the second thread flights (8) and the third thread flights (10) in the direction of the screw shank (3) and in an arc again joins the cutting edge (7) so that barbs (11) are formed which oppose a resistance to a return rotation.

2. A bone screw according to claim 1 **characterised in that** a plurality of recesses (9) are provided in each second thread flight (8) and in each third thread flight (8) on an arc of 360°.

3. A bone screw according to claim 2 **characterised in that** three recesses (9) are provided on an arc of 360°.

4. A bone screw according to one of claims 1 to 3 **characterised in that** the screw shank (3) is of a cylindrical shape.

5. A bone screw according to one of claims 1 to 3 **characterised in that** the screw shank (3) is of a conical shape.

6. A bone screw according to one of claims 1 to 5 **characterised in that** the first thread flights (6) include three thread flights.

7. A bone screw according to one of claims 1 to 6 **characterised in that** the second thread flights (8) include three thread flights.

8. A bone screw according to one of claims 5 to 7 **characterised in that** the length of the cutting edges (7) of the second thread flights (8) is constant.

## Revendications

1. Vis à os, en particulier vis pour os spongieux, qui comporte une tête de vis (2), une tige de vis (3) et un filetage (4) constitué de spires sur la tige de vis (3) avec, partant de l'extrémité libre (5) de la tige de vis (3) et se suivant en direction de la tête de vis (2), un certain nombre de premières spires (6) dont l'arête de coupe (7) est lisse, ces premières spires (6) étant suivies de deuxièmes spires (8) empilées en direction de la tête de vis (2) et présentant sur leurs arêtes de coupe (7) des dégagements (9) qui, en ce qui concerne la longueur de leur arc en direction périphérique, dépassent pour chaque spire celui de la deuxième spire (8) qui la précède en partant de l'extrémité libre (5), un empilage de troisièmes spires (10) présentant des dégagements (9) de même grandeur faisant suite aux deuxièmes spires (9),
cette vis à os étant **caractérisée en ce que** pour chaque deuxième spire (8) et chaque troisième spire (10) le dégagement (9) a la forme d'une corde qui part de l'arête de coupe (7) en direction de la tige de vis (3) et qui revient à cette arête par un arc, en formant ainsi un crochet d'opposition (11) créant une résistance à une rotation de dévissage.

2. Vis à os selon la revendication 1, **caractérisée en ce que** dans chaque deuxième spire (8) et dans chaque troisième spire (10), il est prévu sur un arc de 360° plusieurs dégagements (9).

3. Vis à os selon la revendication 2, **caractérisée en ce que** dans chaque deuxième spire (8) et dans chaque troisième spire (10), il est prévu sur un arc de 360° trois dégagements.

4. Vis à os selon une des revendications 1 à 3, **caractérisée en ce que** la tige de vis (3) est de forme cylindrique.

5. Vis à os selon une des revendications 1 à 3, **caractérisée en ce que** la tige de vis (3) est de forme conique.

6. Vis à os selon une des revendications 1 à 5, **caractérisée en ce que** les premières spires (6) sont au nombre de trois.

7. Vis à os selon une des revendications 1 à 6, **caractérisée en ce que** les deuxièmes spires (8) sont au nombre de trois.

8. Vis à os selon une des revendications 5 à 7, **caractérisée en ce que** la longueur des arêtes de coupe (7) des deuxièmes spires (8) est constante.
